# EUROPEAN PATENT APPLICATION

(11) **EP 0 560 998 A1**
(43) Date of publication of application: **22.09.1993**
(21) Application number: 93906339.2
(22) Date of filing: 08.10.1992
(51) Int. Cl.: A61K 37/02

(54) **MEDICINE FOR PREVENTING AND TREATING BLEEDING TENDENCY**

(30) Priority: 09.10.1991 JP 262354/91
(71) Applicant: TORAY INDUSTRIES, INC., Tokyo 103 (JP)
(72) Inventor: OKAMOTO, Tasuku N102, 31-17, Tsunishi 1-chome, Kanagawa 248 (JP); OKANO, Kiyoshi 4-8-211, Ichinomiya 5-chome, Kanagawa 253-01 (JP); SATO, Yuichiro, Tokyo 146 (JP); IDA, Nobutaka, Kanagawa 248 (JP); NARUTO, Masanobu H-2, 20-31, Tsunishi 1-chome, Kanagawa 248 (JP)
(74) Representative: Kador & Partner
(86) International application number: JP9201309
(87) International publication number: WO9306840

(57) **Abstract**

A medicine for preventing and treating bleeding tendency containing IL-6 as an active ingredient. The administration of IL-6 serves to enhance the functions of both blood platelets and hemangioendothelial cells to thereby remedy hemorrhagic diathesis, thus enhancing the hemostatic function. Thus IL-6 is useful as an excellent medicine for preventing and treating bleeding tendency, since it has an activity of efficiently inhibiting the bleeding tendency of a living organism and is *per se* a physiologically active substance derived from a living organism. Therefore IL-6 is useful in treating various bleeding tendencies, such as one caused by bone marrow inhibition after cancer chemotherapy and radiotherapy, one observed after bone marrow transplantation, one observed in hypoplastic anemia, or one observed in idiopathic thrombocytopenic purpura.

## Description

### [Technical field]

The present invention relates to an agent for preventing or treating hemorrhage tendency. In more detail, the present invention relates to an agent for preventing or treating hemorrhage tendency with interleukin 6 (hereinafter abbreviated as IL-6) as an active ingredient.

### [Background]

Hemorrhage tendency can be generally classified in view of manifestation mechanism into three categories; 〈1〉 quantitative or qualitative abnormality of platelets, 〈2〉 abnormality of blood vessels, and 〈3〉 any one or more as a combination of deficiency or qualitative abnormality or abnormal activation of plasma proteins participating in blood coagulation. Furthermore, the hemorrhage tendency due to these causes may be congenital or acquired. Typical diseases accompanied by hemorrhage tendency include the following very many typical diseases: thrombasthenia such as Bernard-Soulier syndrome and storage pool disease, thrombocytopenia such as Fanconi's syndrome, aplastic anemia, idiopathic thrombocytopenia, drug anaphylaxis, idiopathic thrombocytopenic purpura and disseminated intravascular coagulation (DIC), vascular diseases such as scorbutus and allergic purpura, von Willebrand's disease, vitamin K deficiency, nephrosis syndrome, etc. (Medical Science Dictionary, No. 21, Kodansha, 1982 (in Japanese); Hematology, 4th edition, Williams J.W., Beutler E., Et. al., 1990).

The hemorrhage tendency accompanying the bone marrow suppression after the chemotherapy of cancer or after the radiotherapy of cancer, and the hemorrhage tendency after bone marrow transplantation apparently show the decrease in the number of platelets which is called thrombocytopenia, but the hemorrhage tendency as a clinical dangerous symptom is a comprehensive symptom of combined causes. That is, a chemotherapeutic agent or radiation which induces bone marrow suppression injures the liver and other various organs, to remarkably disorder the homeostasis of the organism, thus inducing hemorrhage tendency as a comprehensive result.

The direct treatment against hemorrhage tendency is achieved by normalizing hemostasis. For example, a typical example of treatment to improve a hemorrhagic predisposition cause due to an abnormal coagulation factor is the treatment of hemophilia. This disease is normal in bleeding time but is longer in coagulation time and partial thromboplastin time, causing hemorrhage in muscles and joints. In this case, the factor VIII or factor XI preparation to compensate the deficiency is administered to normalize hemostasis. Furthermore, a patient suffering from vitamin K deficiency is administered with vitamin K preparation. On the other hand, for the hemorrhage caused by an injured blood vessel wall, a blood vessel reinforcing agent such as flavonoid or ethamsylate is used. However, for the hemorrhage tendency caused by the quantitative or qualitative abnormality of platelets, there is no excellent remedy which can normalize the declining function, and platelet transfusion only is practised. However, the platelet transfusion involves several side effects and problems such as rejection, advent of autoantibody, rapid decrease of transfused platelets and risk of virus infection, and is not a perfect therapy. In this regard, applying a substance inherently existing and functioning as intended in vivo can be said to be desirable since it does not disorder the rhythm of organisms, but among bioactive substances derived from living organisms, no substance is found which can effectively prevent and treat hemorrhage tendency.

On the other hand, interleukin 6 (hereinafter abbreviated as IL-6) has been found to be one bioactive substance with the same molecule, even though it had been studied under different substance names such as interferon β₂ (Zilberstein, A., et. al., EMBO J. 5, 2529-2537, 1986), B-cell stimulating factor 2 (BSF-2) (Hirano, T., et. al., Nature, 324, 73-76, 1986), 26-KDa protein (Hageman, G., et. al., Eur. J. Biochem., 159, 625-632, 1986), hybridoma/plasmacytoma growth factor (VanDamme, J., et. al., J. Exp. Med., 165, 914-919, 1987), hepatic cell stimulating factor (HSF) (Andus, T., et. al., FEBS Lett., 221, 18-22, 1987; Gauldie, J. et. al., Proc. Natl. Acad. Sci. USA, 84, 7251-7255, 1987), etc. The substance was proposed to be called with a single name, IL-6, and the nomenclature has now been established. IL-6 has physiological actions as stated above in connection with respective findings, and in addition, recently, IL-6 is reported to promote the maturation of megakaryocytes in vitro (e.g., Ishibashi, T., et. al., Proc. Natl. Acad. Sci. USA, 86, 5953-57, 1989) and to increase platelets in vivo (e.g., Asano, S., et. al., Blood, 75, 1602-1605, 1990).

As described above, IL-6 is reported to have many physiological activities, but its effect on hemostatic mechanism and coagulation and fibrinolysis is not yet clearly reported. That is, the activity of IL-6 on thrombopoiesis is reported, but its actual effect on the hemostatic mechanism of the organism is not yet known. Since the hemostatic mechanism of IL-6 is not always controlled by the number of platelets only, it is desired to discuss hemostatic ability functionally in distinction from the variation in the number of platelets. That is, simply increasing platelets alone does not always improve the symptom of hemorrhage tendency. On the contrary, if a platelet increasing drug is administered only for increase of platelets, any unwanted side effect may be manifested.

It was reported that the platelets increased in the peripheral blood of IL-6 administered rats are equivalent to those of normal rats in aggregation ability, and that platelets are free from direct action of IL-6 since no IL-6 receptor exists on the plasma membranes of platelets.

Furthermore, it was reported that IL-6 increases factors acting to promote hemostasis and coagulation such as the amount of fibrinogen in blood, and increases blood coagulation control factors like antithrombin III (ATIII), and also increases fibrinolytic factors like plasminogen activator inhibitor (PAI). However, what reactions are actually caused comprehensively by these actions in the in vivo hemostatic mechanism and coagulating and fibrinolytic mechanism remains unknown, and what actions are caused by in vivo administration of IL-6 on in vivo hemostatic ability is far less known.

The present invention intends to purify a bioactive substance which is inherently active at very low concentration in organism, for providing it as a drug. In more detail, hemorrhage tendency is a disease caused by the qualitative or quantitative abnormality of platelets and the abnormality of blood vessel and the deficiency or disorder of coagulation factor. Especially for the hemorrhage tendency caused by any functional disorder of platelets or any disorder of blood vessel, there exists no excellent remedy, and medical services are badly in need of such a remedy. Particularly the hemorrhage tendency accomanying the bone marrow suppression after the chemotherapy of cancer or after the radiotherapy of cancer, the hemorrhage tendency after bone marrow transplantation, or the hemorrhage tendency in aplastic anemia, idiopathic thrombocytopenic purpura, etc. directly endangers the life, and how to control it is an essential therapeutic demand. The object of the present invention is to provide a bioactive substance used for stopping hemorrhage and improving and treating hemorrhage predisposition causes.

### [Disclosure of the invention]

The inventors newly found that IL-6 improves hemorrhage tendency with a more comprehensive and more direct effect than the simple effect of increasing platelets as a parameter, and completed the present invention. The present invention is a prophylactic and therapeutic remedy for hemorrhage tendency with IL-6 as an active ingredient.

The inventors found that in vivo administered IL-6 (1) promotes the functions of platelets, (2) protects and activates vascular endothelial cells, (3) increases factors concerned with platelet aggregation and blood coagulation and fibrinolysis in good balance, and (4) increases platelets for comprehensively intensifying hemostatic ability, thereby allowing hemorrhage tendency to be treated, and present IL-6 as a directly useful hemorrhage tendency prophylactic and therapeutic remedy which has not been presented hitherto.

### [The best embodiment]

The present invention is described below in more detail. The first object in the in vivo administration of IL-6 is not to increase platelets. The present invention has been achieved based on a new finding that the hemorrhage time can be efficiently shortened even if the administration of IL-6 does not increase platelets or only slightly increases platelet number. The prevention and treatment of hemorrhage tendency in the present invention is characterized by simultaneously and comprehensively achieving the qualitative and quantitative functional improvement of platelets, the activation of vascular endothelial cells, the balanced increase of factors concerned with platelet aggregation and blood coagulation and fibrinolysis, and the increase of platelets by IL-6 administration.

The treatment and/or prevention effect of the present invention are obtained partially because the in vivo administration of IL-6 to a mammal (especially a man) promotes platelet adhesion and aggregation, and releasing ability of the factors from platelets described below responsible for intensifying hemostasis. Substances which are contained in the granules of platelets and can be released with activation include, for example, ATP, ADP, serotonin, platelet activating factor (PAF), platelet derived growth factor (PDGF), β-thromboglobulin, platelet factor 4 (PF-4), von Willebrand factor (vWF), thrombospongin, etc. The increase of contents or released amounts of at least some of these substances is an essential factor in the effect of the present invention. Furthermore, the effect of the present invention is estimated to be obtained partially because the in vivo administration of IL-6 intensifies the phospholipid metabolism, calcium ion metabolism, and enzyme metabolism responsible for protein phosphorylation in platelets. That is, it is considered that intracellular single transducing substances such as arachidonic acid, inositol-3-phosphoric acid (IP3), diacylglycerol (DG) and cyclic AMP (cAMP) are promoted to intensify the thrombosis. Moreover, the effect of the present invention is considered to be obtained partially because the in vivo administration of IL-6 causes the increase of surface antigens existing on the plasma membranes of platelets and the intensification of affinity, thereby similarly promoting intercellular single transduction functions for intensifying thrombosis. Such surface antigens include cell adhesion molecules (or receptors) such as GPII_{b}/IIIₐ and GPI_{b}/IX proteins. In addition, the effect of the present invention is considered to be obtained partially because the in vivo administration of IL-6 activates vascular endoehtlial cells, to produce and increase the concentration of von Willebrand factor (vWF) in plasma (in bloodstream), for intensifying the primary hemostasis. Still furthermore, the effect of the present invention is obtained partially because the in vivo administration of IL-6 increases the quantity of factors produced by the liver and concerned with platelet aggregation, blood coagulation and fibrinolysis. The factors concerned with platelet aggregation and blood coagulation and fibrinogenolysis include fibrinogen, ceruloplasmin, antithrombin III and various protease inhibitors. Still moreover, the effect of the present invention is considered to be attributable to the action of IL-6 to increase platelets. In the present invention, it is characteristic that these actions are integrated to intensify hemostatic ability.

As shown in the examples of the present invention described later, the improvement of platelet aggregation and bleeding time by IL-6 is achieved without apparent thrombopoiesis. In this regard, the IL-6 as agent for preventing or treating hemorrhage tendency of the present invention is intended to achieve improvement in all of the normalization or promotion of platelet functions, and the repair and activation of vascular endothelial cells, which could not be achieved by the conventional drugs. The present invention clarifies this fact for the first time.

The IL-6 used in the present invention is not espcially limited, and the mammalian IL-6 which can be obtained by conventional methods can be favorably used. For example, IL-6 obtained by culturing IL-6 producing cells or recombinant IL-6 obtained by gene recombination can be used. For human clinical application, preferably, IL-6 obtained by human IL-6 producing cells can be used.

The IL-6 obtained from cultured human cells can avoid the ingress of impurities derived from any other species than human, and the IL-6 obtained is preferably close to the IL-6 inherently acting in vivo. That is, since the structure containing sugar chains and micro-modifications or processing is close to that of human in vivo IL-6, antibody production can be relatively eliminated when the IL-6 is administered to a man as a drug. Therefore, the IL-6 produced by cultured human cells is expected to be more efficiently effective in vivo.

The IL-6 produced by cultured human cells of the present invention means the IL-6 obtained by culturing the cells derived from a human. More specifically, the IL-6 obtained by culturing normal cells, i.e., adherent type human cells which are not transformed (not extremely transformed) or derived from tumor cells is preferable. The IL-6 obtained under these conditions ordinary has sugar moiety in the structure. As for the normal human cells not derived from cancer cells, especially fibroblasts, endothelial cells, stroma cells, etc. are considered as sources of IL-6 in vivo, and since some of them can be cultured in almost normal cell morphology, they can be especially preferably used. However, the normal human cells to be cultured are not limited to them.

Among the above normal human cells, especially preferable cells are adherent type cells in culture, and so they can be cultured under conventional culturing conditions. A conventional culture flask, roller bottle or micro carrier (fine grains) can be preferably used for culture, but the method is not limited to them. Thus, from the culture medium of the human cells, almost pure IL-6 can be purified through the ordinary steps of purification. Culture methods and purification methods are described in the examples given later, but are not limited thereto or thereby.

On the other hand, recombinant IL-6 can be prepared by known methods. An example using Escherichia coli is stated as one of the examples given later, but any other widely known gene manipulation methods can be used. For example, an IL-6 cDNA can be connected downstream of a promoter, etc. functioning in a host, for introducing as DNA or virus, etc. into prokaryotic cells of Bacillus subtilis, etc., yeast, animal cells such as hamster cells, mouse cells, monkey cells and human cells, insect cells or insect, for preparation of IL-6.

The composition of the present invention contains the human IL-6 prepared according to any of the methods mentioned above, as a main ingredient. Other ingredients can be selected from general pharmaceutical additives. Of course, the object of the present invention can be achieved even without any additive. In general, additives are added mainly for stabilization. Such pharmaceutical additives are selected from proteins and/or saccharides usable as pharmaceutical additives stated in the Pharmacopeia of Japan. Especially preferably, those selected from human serum albumin (HSA), gelatin, mannitol, sorbitol, lactose, torehalose, surfactants, etc. can be used alone or in combination, but the additives are not limited to them.

The present invention also includes the simultaneous or successive administration of IL-6 with other medicines, biological drugs, synthetic drugs, etc. The other medicines and drugs include interleukins such as IL-1, IL-2, IL-3, IL-7 and IL-11, CSF (colony stimulating factor), cytokines such as EPO (erythropoietin), interferon, thrombopoietin, MSF (megakaryocyte stimulating factor) and Meg-CSF (megakaryocyte colony stimulating factor), and other known drugs to complement or assist the object of the present invention of IL-6.

To achieve the object of the present invention to prevent and treat hemorrhage tendency, the composition mainly containing IL-6 thus obtained is administered in vivo. The method for administration is not especially limited. However, for the hemorrhage tendency accompanying the thrombocytopenia after the chemotherapy of cancer, the bone marrow suppression after the radiotherapy of cancer, the bone marrow suppression after bone marrow transplantation, or trauma or operation, etc., proper administration routes selected from intravenous injection, subcutaneous injection, intramuscular injection, intravenous drip infusion, etc. can be used. Permucosal administration such as peroral administration, pernasal administration, perpulmonary administration or perenteral administration can also be effected as the case may be.

The effective dose is selected in a range from 0.0001 to 300 µg/kg/day, preferably 0.001 to 50 µg/kg/day. The dose depends on disease conditions, and is not limited in this range of course. As a feature of the present invention, the dose is usually preferably smaller than the dose showing the thrombopoiesis in vivo, but the present invention is of course not limited to this dose range.

The number of administration times is usually one or twice per day, or one per two or more days, but is not limited in this range.

The preventive and/or therapeutic remedy of the present invention is administered when the patients shows hemorrhage tendency, for example when the number of platelets has decreased, but the timing is not especially limited. As shown in Example 4, remarkable hemorrhage tendency may occur even if the decrease of platelet is slight, and even in such a case, the drug of the present invention is effective in imporoving the hemorrhage tendency.

The serious thrombocytopenia in human clinic is often defined as a condition that the number of platelets is less than 20,000 cells/µl, and platelet transfusion is usually practiced for such serious thrombocytopenia. However, the hemorrhage tendency attributable to the administration of a chemotherapeutic agent or any other pathogenic predisposition is not always correlated with decrease the number of platelets to less than 20,000 cells/µl. In most of such cases, the composition containing IL-6 of the present invention can be effectively used. Medium thrombocytopenia is defined as less than 40,000 ∼ 50,000 cells/µl in the number of platelets, and usually platelet transfusion is not practiced. However, if the patient has hemorrhage tendency and is feared to suffer internal hemorrhage, he can be treated by the composition of the present invention. If hemorrhage tendency attributable to the administration of a chemotherapeutic agent or any other cause occurs or if the occurrence of hemorrhage tendency is predicted beforehand, with the number of platelets kept at more than 40,000 ∼ 50,000 cells/µl, the composition of the present invention can be used for treatment or prevention.

As a feature of the present invention, the dose can be smaller than that used to cause in vivo thrombocytosis, and the number of administration times is not required to be so may as to cause thrombocytosis, to achieve the usefulness of the hemorrhage tendency preventive remedy of the present invention. However, the present invention is not limited to this dose range or this number of administration times.

### [Examples]

The present invention is described below in more detail and concretely in reference to examples, but is not limited thereto or thereby.

The activity of IL-6 was evaluated according to the following methods.

### Bioactivity assay method:

Established cell line 7TD1 (IL-6 dependent hybridomas (J. van Snick, et al., European J. Immunol., 18, 193-197 (1988)) was used, and a proper amount of IL-6 was added to it. The cell proliferation of 7TD1 was measured by MTT method. Separately stepwise diluted samples of standard IL-6 were prepared, and in comparison with their proliferation activities, the bioactivity of the IL-6 obtaied was evaluated. The standard IL-6 used was the same as that attached to the human IL-6 ELISA kit produced by Toray Industries, Inc. shown below.

### ELISA (enzymelinked immunosorbent assay)

ELISA using IL-6 antibody (N. Ida, et al., Biochem. Biophys. Res. Commun., 165, 728-734, (1989)) was used for measurement. The human IL-6 ELISA kit produced by Toray Industries, Inc. and sold by Toray Fujibionics was used to evaluate IL-6.

### Example 1

### Preparation of Escherichia coli-derived IL-6:

An IL-6 expression vector which has IL-6 cDNA with the same gene sequence as stated in a published report (T. Hirano, et al., Nature, vol. 324, 73 (1986)) was prepared according to the following method.

Cell line NIM-1 cells derived from thyroid gland cancer (Kaoru Toyama, et al., Journal of Japanese Society of Hematology, vol. 53, 805 (1990)) were cultured, to prepare mRNA by an ordinary method, and from it, cDNA mixture was synthesized by reverse transcriptase. It was subjected to PCR reaction with the following two DNA oligomers as primers:
CCGATCGATGCCAGTACCCCCAGGA (sequence No. 1 in sequence list)
and
GCCACGGATCCTACATTTGCCGAAG (sequence No. 2 in sequence list)
The obtained amplified DNA was digested by restriction enzymes ClaI and BamHI, and the obtained DNA fragment was inserted between the ClaI region and BglII region of Escherichia coli expression vector pKM6 (Tanaka, et al., J. Interferon Res., 6, 429-35 (1986)), to obtain expression IL-6 vector pKMIL-6. The pKMIL-6 was introduced into Escherichia coli HB101, to obtain a recombinant. The reocmbinant was cultured as follows, to prepare Escherichia coli recombinant IL-6.

Escherichia coli HB101/pKMIL-6 with human interleukin-6 expression palsmid was cultured using a 30-liter jar. Thirty liters of a growing medium (containing 0.3% of potassium dihydrogenphosphate, 0.6% of disodium hydrogenphosphate, 0.5% of sodium chloride, 0.1% of ammonium chloride, 0.5% of glucose, 0.5% of Casamino acid, 1 mM of magnesium sulfate, 3 µM of ferrous sulfate, 6 µg/ml of vitamin B1 and 50 µg/ml of ampicillin) was supplied into the 30-liter jar. Said recombinant was planted there. The jar was operated at a speed of 300 rpm with air of 1 VVM at 25°C. Indoleacrylic acid as a substance to induce tryptophan operon was added, and while gluclose and Casamino acid were added, culture was effected for 60 hours. The cultured biomass was collected by centrifugation at 10,000 × g for 20 minutes. The amount of the biomass obtained was about 895 g. The collected biomass was suspended in 50 mM Tris hydrochloride buffer of pH 8.0 containing 1 mM of EDTA and 100 mM of NaCl, to achieve optical density 20 at 550 nm. The biomass was crushed and centrifuged, and the extract was recovered. The extract was 235 g in protein content and 495 mg in interleukin-6 content. The amount of IL-6 was measured by said ELISA (hereinafter this applies).

The extract was adsorbed by 5.5 liters of silica in a column, and eluted by an acid solution. The recovery of IL-6 was 462 mg. To the eluate, ammonium sulfate was added to achieve a final concentration of 1.33 M, and insoluble impurities were removed by centrifugation. The supernatant was adsorbed by 200 ml of a butyl column (Butyl Toyopearl produced by Tosoh Corp.), and eluted by a low salt neutral solution. 237 mg of IL-6 of 84% purity (SDS-PAGE) was obtained. The eluted IL-6 was adsorbed by 80 ml of a hepain column (AF Heparin Toyopearl produced by Tosoh Corp). IL-6 was eluted by a neutral salt buffer, resulting in 114 mg of IL-6 of 91% in purity. The eluate was purified again by 200 ml of a butyl column (Butyl Toyopearl produced by Tosoh corp), to obtain 66 mg of IL-6. The purity of the IL-6 prepared as above was found to be more than 95% by reverse phase HPLC. Said IL-6 was confirmed to have activity, by the assay method stated above.

### Example 2

### Preparation of human cell-derived IL-6:

One example of the IL-6 of the present invention was prepared according to the following method.

Human fibroblasts were cultured on beads in suspension culture in 1-liter Eagle's minimum essential medium containing 5% NCS (Newborn Carb Serum) in a 2-liter glass culture tank to obtain 10⁶ cells per milliliter (beads: "Cytodex 1" produced by Pharmacia, 37°C). Then, the medium was replaced by 1 liter of a serum-free Eagle's minimum essential medium containing a small amount of carboxymethyl cellulose, and 100,000 units/l of human natural interferon β was added as priming agent. The next day, furthermore, 50 mg/l of poly I : poly C and 10 mg/l of cycloheximide were added. Four hours later, 4 mg/l of actinomycin D was added, and further 1 hour later, the producing medium was replaced by an Eagle's minimum essential medium containing a small amount of methyl cellulose, to effect super-induction treatment. Subsequently, for 2 days, the culture was continued at 37°C.

Stirring was stopped, causing the micro-carrier to settle, and the supernatant solution and the washing from the producing medium were filtered. One liter of the filtrate was put into another container with a stirrer. Into the produced liquid, sterilized "Blue Sepharose CL-6BFF" (produced by Pharmacia) was added. The mixture was stirred at 15°C for 4 days, for batch adsorption. The stirring was stopped, causing the blue carrier to settle, and the supernatant solution was put into another container. Silica carrier sterilized by high pressure steam at 121°C for 30 minutes in sodium phosphate buffer was packed into two columns by 4 ml each, which were connected in series. Through the columns, the supernatant solution obtained through the blue carrier was passed at a flow rate of 20 ml/hr. After passing all the amount, the adsorbed IL-6 was purified dependently colum by colum in separate experiments. Through each of the columns, 25 ml of sodium phosphate buffer was passed, and subseqeutnly 20 mM hydrochloric acid was passed, to recover 10 ml of fractions containing IL-6. To the recovered hydrochloric acid solution, ammonium sulfate was further added, to achieve 1.33 M, and the mixture was stirred gently at 4°C onvernight. The precipitate was removed by centrifugation at 3000 rpm at 4°C for 30 minutes.

The separated supernatant solution was passed through a column packed with 1ml of a hydrophobic chromatography carrier (Butyl Toyopearl 650M produced by Tosoh Corp), for adsorption. The column was washed with 20 mM hydrochloric acid containing 1.33 M of ammonium sulfate and 50 mM sodium phosphate buffer containing 1.3 M of ammonium sulfate, and then 50 mM sodium phosphate buffer was used for recovery. Subsequently, high performance liquid chromatography (Shimadzu LC-4A) equipped with ODS column (C₁₈) (YMC-Pack ODS A-312 S-5 120A produced by YMC) was used for purification. Reverse phase chromatography with gradient elution by water containing 0.1% of trifluoroacetic acid and acetonitrile containing 0.1% of trifluoroacetic acid was performed to fractionate human natural interleukin-6. The human natural interleukin-6 thus obtained was gel-filtered by "Sephadex G-25 (produced by Pharmacia) with 5 mM of formic acid as a solvent to obtain interleukin-6 solution not containing acetonitrile.

The purity of the IL-6 prepared as above was found to be more than 95% by reverse phase HPLC. Said IL-6 was confirmed to have activity by said assay method.

### Example 3

### Promotion of hemostasis by IL-6:

C57BL/6 mice were subcutaneously administered with the IL-6 solution obtained in Example 2 every day once per day, and on the 11th day, the bleeding time was measured. As a control group, mice were treated with physiological saline in the same manner. For measurement of bleeding time, the tail of each mouse was cut by 1 cm apart from the tip, and the blood flow was infiltrated into a filter paper every 5 seconds spot by spot. The time when no bleeding spot was observed any more was measured. The dose of IL-6 was 280 µg/kg/day.

As a result, the control group (n = 8) administred with physiological saline was 100.0±6.7 seconds in mean bleeding time (mean bleeding time ± SE), but the IL-6 administered group (n = 8) was 54.7±5.4 seconds in mean bleeding time, to show statistically significant (p < 0.005) shortening of bleeding time. In this case, the number of platelets in the IL-6 administered group increased to about 135% of that of the physiological saline administered group (designated as 100%), showing an increase of only about 35%. So, it can be said that the reaction for shortening the bleeding time (time taken till hemostasis is achieved) observed in the in vivo adminstration of IL-6 is sharper than the reaction of thrombocytosis.

Similarly, IL-6 was subcutaneously administered once per day, and the bleeding time was measured on day 2 after twice administration. As a result, the control group administered with physiological saline was 110.5±5.4 seconds in mean bleeding time, while the IL-6 twice administered group was 80.5±7.7 seconds in mean bleeding time, to show statistically significant (p < 0.01) shortening by only twice administration of IL-6. In this case, the number of platelets in the IL-6 administered group was not significantly different from that of the control group. The above results clarified the promotion of hemostasis by IL-6 without any correlation with thrombocytosis.

### Example 4

### (1) Recovery of bleeding time in a chemotherapeutic agent-administered mice model (subcutaneous administration):

C57BL/6 mice were treated with single intraperitoneal administration of 2 mg/kg of mitomycin C (hereinafter written as MMC) as a chemotherapeutic agent for cancer, to induce the thrombocytopenia. The mice were subcutaneously administered with the IL-6 prepared in Example 2 for 11 days once per day from the 7th day after MMC administration when platelets began to decrease, and the bleeding time was measured on the day following the final administration day as done in Example 3. The dose of IL-6 was 280 µg/kg/day.

As a result, the control group treated with repeating administraions of physiological saline alone after having been administered with MMC (platelet decreased group) exhibited 131.4±15.1 seconds in mean bleeding time, with statistically sgnificant (0 < 0.01) delay, compared to the normal mice group of 100.9±6.7 seconds in mean bleeding time. On the other hand, the group treated with repeating continuous administrations of IL-6 after having been administered with MMC (platelets recovered group) exhibited 82.7±6.6 in mean bleeding time, with statistically significant (p < 0.01) shortening compared to the MMC only administered group. In this case, the MMC administered mice were decreased in the number of platelets only to about 70% of the normal value (100%), but were remarkable in hemorrhage tendency, with deleterious sighn in the general condition. In this example, the administered chemotherapeutic agent injured the liver and other organs, to induce more general hemorrhage tendency, and even though thrombocytopenia was slight, hemorrhage tendency was remarkable. Even in such a case, IL-6 was effective in improving hemorrhage tendency, to show that the increase of platelets was not the main action. That is, the time required to produce hemostasis was achieved was as long as 131.4 seconds with the control group administered with MMC but not administered with IL-6, but the mean bleeding time of the group administered with MMC and further with IL-6 was as, short as 82.7 seconds. The control group administered with physiological saline without having been administered with MMC recorded 100.9 seconds as mean time (the control group showing the normal value). In this case, the IL-6 administration shortened the bleeding time beyond the normal value. On the other hand, the number of platelets was recovered only to 97% of the noarmal value (termed as 100%) in this case. This shows that IL-6 is more effectively act on the improvement of hemorrhage predisposition cause (treatment and/or prevention) than on thrombocytosis.

### (2) Recovery of bleeding time in a chemotherapeutic agent administered mice model (intravenous administration):

C57BL/6 mice were intraperitoneally administered once with 2 mg/kg of MMC as a chemotherapeutic agent for cancer, to induce thrombocytopenia. The mice were daily intravenously administered with IL-6 from the tail vein for 6 days once per day from the 7th day after the MMC administration when platelets began to decrease, and the bleeding time was measured on the day following the final administration day as done in Example 3. The dose of IL-6 gas 177 µg/kg/day. As a result, the mean bleeding time of the control group administered in combination with MMC and repeating physiological saline showed 151.4±15.1 seconds, showing statistically significant (p < 0.01) delay, compared to the mean bleeding time of 130.9±6.7 seconds recorded by the normal mice group. On the other hand, the group administered with MMC and then continuously with IL-6 showed 134.7±6.6 seconds in mean bleeding time which was statistically significantly (p < 0.01) shorter than that of the group administered with MMC only, to show the effect of recovering to the normal value.

From the above results, it was found that the mice having prolonged bleeding time caused by chemotherapeutic agent treatment were improved to normal bleeding time with IL-6 treatment.

### Example 5

### Promotion of the adhesion and aggregation activity on collagen stimulated platelets by IL-6:

C57BL/6 mice (n = 8) were intraperitoneally administered once with 2 mg/kg of MMC as a chemotherapeutic agent for cancer. The mice was repeatedly intravenously administered from the tail vein with the IL-6 prepared in Example 2 for 6 days once per day from the 7th day after MMC administration when platelets began to decrease, and the platelet adhesion and aggregation ability was measured on the day following the final administration day. The dose of IL-6 was 177 µg/kg/day. To describe in detail, on the day following the completion of the repeating IL-6 administration, platelet rich plasma (PRP) was prepared. With 2 µg/ml of collagen used as an aggregating agent, the adhesion and aggregation activity was measured by an impedance aggregometer (Whole-Blood Aggro Meter Type 560, CHRONOLOG. Corp.) For the PRP, the number of platelets was adjusted to 80 × 10⁴ cells/µl. As a result, the maximum extension in impedance (10-minute value) evoked by the platelets taken from the IL-6 administered mice was 7.5 Ω, being higher than 4.8 Ω of the platelets of the control mice similarly administered with physiological saline. These results and the results of Example 6 described below suggest that in the animals administered with IL-6, the phospholipid-arachidonic acid metabolism of their platelets is enhanced, resulting in the increase thromboxane A2 production and the quantity of free Ca²⁺, and thus it was found that finally the adhesion and aggregation ability of the platelets is intensified.

### Example 6

### Promotion of the ATP release activity of collagen stimulated platelets by IL-6:

C57BL/6 mice (n = 8) were intraperitoneally administered once with 2 mg/kg of MMC as a chemotherapeutic agent for cancer. The mice were repeatedly intavenously administered with IL-6 from the tail vein for 6 days once per day from the 7th day after the MMC administration when platelets began to decrease, and the releasability of ATP from platelets was measured. The dose of IL-6 was 177 µg/kg/day. To describe in detail, after completion of IL-6 administration, platelet rich plasma (PRP) was prepared, and 2 µg/ml of collagen was used to elicite adhesion and aggregation. The amount of ATP released in this case was determined by the fluorometer (Whole-Blood Aggro Meter Type 560, CHRONOLOG. Corp.) using luciferin·luciferase reagent. As a result, the maximum amount of released ATP of the platelets taken from the IL-6 administered mice was 1.6±0.2 µM, being larger than 1.2±0.1 µM of the platelets of the control mice similarly administered with physiological saline. These results suggest that in the animals administered with IL-6, the phospholipid arachidonic acid metabolism of theirs platelets is enhanced, to increase thromboxane A2 production and the quantity of free Ca²⁺, increasing the subsequently occurring ATP release reaction, and thus it was found that finally the adhesion and aggregation ability of the platelets is intensified. The other known substances released by platelets except for ATP typically include ADP, serotonin, platelet activating factor (PAF), platelet factor 4 (PF4), von Willebrand factor (VWF), thrombospongin, etc. Among them, ATP was measured as an indicator in this experiment.

### Example 7

### Promotion of the adhesion and aggregation activity of arachidonic acid stimulated platelets by IL-6:

C57BL/6 mice (n = 8) were intraperitoneally administered once with 2 mg/kg of MMC as a chemotherapeutic agent for cancer. The mice were continuously intraveneously administered with the IL-6 prepared in Example 2 at the tail for 6 days once per day from the 7th day after the MMC administration when platelets began to decrease, and the adhesion and aggregation ability of platelets was measured on the following day of the final administration day. The dose of IL-6 was 177 µg/kg/day. To describe in detail, after completion of IL-6 administration, PRP was prepared, and with 400 µM of arachidonic acid used as an agrregating agent, the adhesion and aggregation activity was measured by an impedance aggregometer (Whole-Blood Agrro Meter Type 560, CHRONOLOG. Corp.) For the PRP, the number of platelets was adjusted to 80 × 10⁴ cells/µl. As a result, the platelets taken from the IL-6 administered mice recorded a maximum extension in impedance of 18.7 Ω at 7.7 minutes after the induction, higher than 15.0 Ω recorded by the control mice similarly administered with physiological saline at 9.9 minutes after the induction. These results suggest that in the animals administered with IL-6, the arachidonic acid metabolism of their platelets is enhanced, to increase thromboxane A2 production and the quantity of free Ca²⁺, and thus it was found that finally the adhesion and aggregation ability of the platelets is intensified.

### Example 8

### Promotion of platelet aggregation activity by IL-6 (rats):

An examination as done in Example 3 was performed using rat as the animal species. That is, Sprague-Dawley (SD) male rats of 8 weeks old (about 250 g) were subcutaneously administered with 0.5 ml/body of IL-6 solution (100 µg/ml) every day once per day, and the platelet aggregation was measured on the 6th day. The control solution used was physiological saline. The platelet aggregation activity was measured as follows.

Five milliliters of citrate blood was centrifuged at 1,000 rpm at room temperature for 10 minutes, and the upper layer (PRP: platelet rich plasma) was taken. Furthermore, the lower layer was centrifuged at 3,000 rpm at room temperature for 20 minutes, and the resulting upper layer (PPP: platelet poor plasma) was separated. The PRP was diluted by the PPP, to achieve a platelet concentration of 5 x 10⁵ cells/µl. To 200 µl of the platelet suspension, ADP or collagen was added to achieve a final concentration of 3 µM or 10 µg/ml respectively, and the maximum aggregation rate(%) was measured. The aggregometer used was Hematracer Model 801 produced by Niko Bioscience according to the optical method.

As a result, the physiological saline administered group (n = 4) with ADP added was 32.8±2.7% (mean value ± SE) in platelet aggregation response and that with collagen added was 12.8±4.9%. On the other hand, the IL-6 administered group (n = 3) was 48.5±0.5% (ADP) or 47.0±3.0% (collagen) respectively, to show statistically significant (p < 0.05) increase. In this case, the number of platelet in peripheral blood in the IL-6 administered group was 148.8±4.3 × 10⁴ pieces/µl, showing a significantly high (p < 0.001) value compared to that in the physiological saline administered group (94.4±1.6 × 10⁴ pieces/µl).

The above results clarified that at the stage when the number of platelets was increased by IL-6 administration, the aggregation ability per unit number of platelets was promoted as in Example 3.

### Example 9

### Promotion of platelet aggregation activity in a chemotherapeutic agent administered model (rats):

An examination as done in Example 4 was performed using rats as the animal species. That is, Sprague-Dawley (SD) male rats of 8 weeks old (about 250 g) were intravenousy administered once with 22.5 mg/kg of nimustine hydrochloride (hereinafter written as ACNU) as a chemotherapeutic agent for cancer. The rats were subcutaneously administered with 0.5 ml of IL-6 solution (100 µg/ml) for 5 days every day once per day from the following day of ACNU administration, and on the 5th day, the platelet aggregation ability by the transmittance method was measured as done in Example 8. The control solution used was physiological saline. The platelet aggregation ability was measured with the final collagen concentration at 20 µg/ml.

As a result, the physiological saline administered group (n = 4) was 44.3±6.8% (mean value ± SE) in platelet aggregation rate, while the IL-6 administered group (n = 4) was 52.3±4.9%, to show statistically significant (p < 0.01) increase. Furthermore, in this case, the number of platelets was decreased to 78% (89.7±4.5 × 10⁴ cells/µl) of the normal value (114.4±7.3 × 10⁴ cells/µl) by ACNU administration, but the number of platelets of the group administered with IL-6 after having been administered with ACNU was 114.0±11.1 × 10⁴ cells/µl, almost the same as the normal value.

The above results clarified that the in the stage when the decrease of platelets could be inhibited by IL-6 administration, the platelets decreased model of chemotherapeutic agent administered rats was promoted in platelet aggregation ability per unit number of platelets.

### Example 10

### Increase of vWF (von Willebrand factor) by IL-6:

C57BL/6 mice (n = 8) were intraperitoneally administered once with 2 mg/kg of MMC as a chemotherapeutic agent for cancer. The mice were repeatedly intravenously administered at the tail vein with the IL-6 prepared in Example 2 for 6 days once per day from the 7th day after the MMC administration when platelets began to decrease, and on the following day of the final administration day, the free von Willebrand factor (vWF) concentration in the plasma was measured according to the sandwich EIA method. The dose of IL-6 was 177 µg /kg/day. As a result, the mice administered with MMC only was 3.8±0.3 µg/ml in the vWF concentration in plasma, showing significant (p < 0.01) decrease compared to 4.7±0.6 µg/ml of the control mice administered with physiological saline. On the contrary, the mice administered with MMC and then repeatedly with IL-6 were 4.4±0.4 µg/ml, significantly (p < 0.01) higher than the mice administered with MMC only, to reach the normal value. From these results, it can be estimated that in the animals administered with IL-6, vascular endothelial cells are activated to raise the abibility of producing and releasing some proteins, and at least a fact was indicated, that the free vWF concentration in blood was increased.

### Example 11

### Action of IL-6 to intensify vWF production of cultured human vascular endoehtlial cells:

From the umbilical cord veins of a healthy person, vascular endothelial cells were separated according to a conventional method and cultured in vitro. The culture was performed using RPMI-1640 medium containing 10% of fetal calf serum. With a 24-multiwell plate used, endothelial cells were added by 5 × 10⁵ cells/well, and culture was done at 37°C in an atmosphere of 5% CO₂ and 95% air. To the endothelial cells of a group (n = 3), the IL-6 prepared in Example 2 was added by 10 ng/ml, and to a control group (n = 3), physiological saline was added by the same quantity. Both were cultured for 48 hours. After completion of culture, the supernatant solution was recovered, and the quantity of vWF contained was determined by the sandwich EIA method as done in Example 10. As a result, the quantity of vWF in the IL-6 added endothelial cells was 400±45 ng/ml, significantly (p < 0.01) larger than 280±35 ng/ml of the control group. From the result, it can be seen that IL-6 acts on human vascular endothelial cells, to activate them, for increasing the production of vWF.

### Example 12

### Action of IL-6 to increase fibrinogen production:

C57BL/6 mice (n = 8) were intraperitoneally administered once with 2 mg/kg of MMC as a chemotherapeutic agent for cancer. The mice were repeatedly intravenously administered from the tail vein with the IL-6 prepared in Example 2 for 6 days once per day, and the free fibrinogen (hereinafter abbreviated as Fbg) concentration in the plasma was measured. The dose of IL-6 was 177 µg/kg/day. As a result, the mice administered with MMC only were 140.4±8.0 mg/dl in the Fbg concentration in plasma, to record a significantly (p < 0.01) lower value compared to 211.9±22.6 mg/dl of the control mice administered with physiological saline. On the contrary, the mice administered with MMC and then repeatedly with IL-6 were 239.3±7.3 mg/dl, recording a significantly (p < 0.01) higher value than the group administered with MMC only, to reach the normal value. Furthermore, also when the dose of IL-6 per day was decreased to 1.77 µg/kg/day, the Fbg concentration in plasma was 191.8±5.6 mg/dl, being significantly (p < 0.01) higher than that of the group administered with MMC only, to reach the normal value. From these results, it can be seen that in the blood of animals administered with IL-6, the concentration of the free Fbg which can be produced by the liver increases.

### Example 13

### Normalization of hemorrhage time by IL-6:

The same experimental groups as used in Example 5 were used and the same hemorrhage time measuring method as used in Example 3 was used. As a result, the group administered with MMC only (n = 8) was 155±9 seconds in mean hemorrhage time, recording a statistically significantly (p < 0.001) longer time compared to the mean hemorrhage time of 130±10 seconds recorded by the physiological salt solution administered group (n = 8). It can be said that the MMC administered mice manifested hemorrhage tendency.

On the contrary, the group administered with MMC and then with 1.77 µg/kg/day continuously 6 times (n = 8) was 145±14 seconds in mean hemorrhage time (p < 0.21); the group administered with 17.7 µg/kg/day (n = 8), 135±3 seconds (p < 0.001); and furthermore the group administered with 177 µg/kg/day (n = 8), 133±5 seconds (p < 0.001), recording statistically significant shorter times, to show recovery of normal hemorrhage time. From the results, it can be seen that IL-6 can be used to improve hemorrhage tendency, for normalizing hemorrhage time.

### [Industrial applicability]

As described above, the present invention can provide a drug which can improve in vivo predispositions to bleeding disorder, to prevent or treat hemorrhage tendency. That is, IL-6 is a new prophylactic or therapeutical remedy which can comprehensively improve the hemorrhage tendency caused by the qualitative and quantitative abnormality of platelets and the abnormality of blood vessels, to normalize the hemostatic mechanism, and no excellent prophylactic or therapeutical remedy has been available hitherto for such hemorrhage tendency. IL-6 is effective for treating especially the hemorrhage tendency attributable to the bone marrow suppression occurring after the chemotherapy of cancer or after the radiotherapy of cancer, the hemorrhage tendency occurring after bone marrow transplantation, the hemorrhage tendency attributable to aplastic anemia, and the hemorrhage tendency in idiopathic thromocytopenic purpura, etc. Furthermore, since IL-6 exists in vivo, an excellent phamaceutical composition for treating or preventing hemorrhage tendency more compatible with organisms compared with synthetic drugs can be presented.

Sequence list
Sequence number: 1
Sequence length: 25
Sequence type: Nucleic acid
Number of chains: One
Topology: Straight chain
Kind of sequence: Another nucleic acid synthesized DNA
Sequence: CCGATCGATG CCAGTACCCC CAGGA
Sequence number: 2
Sequence length: 25
Type of sequence: Nucleic acid
Number of chains: One
Topology: Straight chain
Kind of sequence: Another nucleic acid synthesized DNA
Sequence: GCCAGGGATC CTACATTTGC CGAAG

## Claims

1. An agent for preventing or treating hemorrhage tendency, comprising interleukin 6 as an active ingredient.

2. An agent for preventing or treating hemorrhage tendency, according to claim 1, wherein interleukin 6 is produced by cultured human cells.
